# EUROPEAN PATENT APPLICATION

(11) **EP 1 508 615 A1**
(43) Date of publication of application: **23.02.2005**
(21) Application number: 03077602.5
(22) Date of filing: 18.08.2003
(51) Int. Cl.: C12N 7/00, C07K 14/165, A61K 39/215, C12Q 1/68, G01N 33/569, C07K 16/10

(54) **Coronavirus, nucleic acid, protein, and methods for the generation of vaccine, medicaments and diagnostics**

(71) Applicant: Amsterdam Institute of Viral Genomics B.V., 1105 BA Amsterdam (NL)
(72) Inventor: van der Hoek, Cornelia, 1111 HK Diemen (NL)
(74) Representative: Prins, Adrianus Willem, Mr. Ir.

(57) **Abstract**

A new coronavirus (human coronavirus NL63 (HCoV-NL63)) is disclosed herein with a tropism that includes humans. Means and methods are provided for diagnosing subjects (previously) infected with the virus. Also provided are among others vaccines, medicaments, nucleic acids and specific binding members.

SEQUENCE LISTING

<110> PrimaGen Holding B.V.

<120> Coronavirus, nucleic acid, protein, and methods for the
      generation of vaccine, medicaments and diagnostics.

<130> P65647EP00

<140> 03077602.5
<141> 2003-08-18

<160> 27

<170> PatentIn Ver. 2.1

<210> 1
<211> 17
<212> DNA
<213> Artificial Sequence

<220>
<223> Description of Artificial Sequence: top strand MSE
      adaptor

<220>
<221> misc_feature
<222> (1)..(17)

<400> 1
ctcgtagact gcgtacc                                                17


<210> 2
<211> 16
<212> DNA
<213> Artificial Sequence

<220>
<223> Description of Artificial Sequence: top strand for
      HinP1 adaptor

<220>
<221> misc_feature
<222> (1)..(16)

<400> 2
gacgatgagt cctgac                                                 16


<210> 3
<211> 13
<212> DNA
<213> Artificial Sequence

<220>
<223> Description of Artificial Sequence: bottom strand
      oligo for MSE adaptor

<220>
<221> misc_feature

<222> (1)..(13)

<400> 3
taggtacgca gtc                                                          13


<210> 4
<211> 14
<212> DNA
<213> Artificial Sequence

<220>
<223> Description of Artificial Sequence: bottom strand
      oligo for HinP1 adaptor

<220>
<221> misc_feature
<222> (1)..(14)

<400> 4
cggtcaggac tcat                                                         14


<210> 5
<211> 19
<212> DNA
<213> Artificial Sequence

<220>
<223> Description of Artificial Sequence: HinPI standard
      primer

<220>
<221> misc_feature
<222> (1)..(19)

<400> 5
gacgatgagt cctgaccgc                                                    19


<210> 6
<211> 20
<212> DNA
<213> Artificial Sequence

<220>
<223> Description of Artificial Sequence: MseI standard
      primer

<220>
<221> misc_feature
<222> (1)..(20)

<400> 6
ctcgtagact gcgtacctaa                                                   20


<210> 7
<211> 24
<212> DNA
<213> Artificial Sequence

<220>
<223> Description of Artificial Sequence:
      oligonucleotide J2H2R

<220>
<221> misc_feature
<222> (1)..(24)

<400> 7
gctatcatca caatggacnn nnng                                              24


<210> 8
<211> 129
<212> DNA
<213> Human coronavirus

<220>
<223> Description of Combined DNA/RNA Molecule: part of
      replicase polyprotein 1ab

<220>
<221> CDS
<222> (3)..(128)

<400> 8
gt att gtt ttt gtt gct tgt gcc cat gct gct gtt gat tcc tta tgt     47
   Ile Val Phe Val Ala Cys Ala His Ala Ala Val Asp Ser Leu Cys
     1               5                  10                  15

gca aaa gct atg act gtt tat agc att gat aag tgt act agg att ata     95
Ala Lys Ala Met Thr Val Tyr Ser Ile Asp Lys Cys Thr Arg Ile Ile
                20                  25                  30

cct gca aga gct cgg gtt gag tgt tat agt ggc t                      129
Pro Ala Arg Ala Arg Val Glu Cys Tyr Ser Gly
            35                  40


<210> 9
<211> 42
<212> PRT
<213> Human coronavirus
<223> Description of Combined DNA/RNA Molecule: part of
      replicase polyprotein 1ab

<400> 9
Ile Val Phe Val Ala Cys Ala His Ala Ala Val Asp Ser Leu Cys Ala
  1               5                  10                  15

Lys Ala Met Thr Val Tyr Ser Ile Asp Lys Cys Thr Arg Ile Ile Pro
            20                  25                  30

Ala Arg Ala Arg Val Glu Cys Tyr Ser Gly
        35                  40


<210> 10
<211> 145

<212> DNA
<213> Human coronavirus

<220>
<223> Description of Combined DNA/RNA Molecule: part of
      spike protein

<220>
<221> CDS
<222> (3)..(143)

<400> 10
at ggg tct aga tat ggc ttg caa aac tta cta cag tta cct aac ttt     47
   Gly Ser Arg Tyr Gly Leu Gln Asn Leu Leu Gln Leu Pro Asn Phe
     1               5                  10                  15

tat tat gtt agt aat ggt ggt aac aat tgc act acg gcc gtt atg acc    95
Tyr Tyr Val Ser Asn Gly Gly Asn Asn Cys Thr Thr Ala Val Met Thr
                20                  25                  30

tat tct aat ttt ggt att tgt gct gat ggt tct ttg att cct gtt cgt    143
Tyr Ser Asn Phe Gly Ile Cys Ala Asp Gly Ser Leu Ile Pro Val Arg
            35                  40                  45

cc                                                                 145


<210> 11
<211> 47
<212> PRT
<213> Human coronavirus
<223> Description of Combined DNA/RNA Molecule: part of
      spike protein

<400> 11
Gly Ser Arg Tyr Gly Leu Gln Asn Leu Leu Gln Leu Pro Asn Phe Tyr
  1               5                  10                  15

Tyr Val Ser Asn Gly Gly Asn Asn Cys Thr Thr Ala Val Met Thr Tyr
            20                  25                  30

Ser Asn Phe Gly Ile Cys Ala Asp Gly Ser Leu Ile Pro Val Arg
        35                  40                  45



<210> 12
<211> 71
<212> DNA
<213> Human coronavirus

<220>
<221> misc_feature
<222> (1)..(71)
<223> /note="Part of 3'-UTR"

<400> 12
atgataaggg tttagtctta cacacaatgg taggccagtg atagtaaagt gtaagtaatt 60
tgctatcata t                                                      71

<210> 13
<211> 134
<212> DNA
<213> Human coronavirus

<220>
<223> Description of Combined DNA/RNA Molecule: part of
      replicase polyprotein lab

<220>
<221> CDS
<222> (3)..(134)

<400> 13
at gtc agt gat gca tat gct aat ttg gtt cca tat tac caa ctt att     47
   Val Ser Asp Ala Tyr Ala Asn Leu Val Pro Tyr Tyr Gln Leu Ile
     1               5                  10                  15

ggt aaa caa aag ata act aca ata cag ggt cct cct ggt agt ggt aag    95
Gly Lys Gln Lys Ile Thr Thr Ile Gln Gly Pro Pro Gly Ser Gly Lys
                20                  25                  30

tca cat tgt tcc att gga ctt gga ttg tac tac cca ggt                134
Ser His Cys Ser Ile Gly Leu Gly Leu Tyr Tyr Pro Gly
            35                  40


<210> 14
<211> 44
<212> PRT
<213> Human coronavirus
<223> Description of Combined DNA/RNA Molecule: part of
      replicase polyprotein lab

<400> 14
Val Ser Asp Ala Tyr Ala Asn Leu Val Pro Tyr Tyr Gln Leu Ile Gly
  1               5                  10                  15

Lys Gln Lys Ile Thr Thr Ile Gln Gly Pro Pro Gly Ser Gly Lys Ser
            20                  25                  30

His Cys Ser Ile Gly Leu Gly Leu Tyr Tyr Pro Gly
        35                  40


<210> 15
<211> 207
<212> DNA
<213> Human coronavirus

<220>
<223> Description of Combined DNA/RNA Molecule: part of
      replicase polyprotein lab

<220>
<221> CDS
<222> (2)..(205)

<400> 15
a tct aaa cta aac aaa atg gct agt gta aat tgg gcc gat gac aga gct 49

Ser Lys Leu Asn Lys Met Ala Ser Val Asn Trp Ala Asp Asp Arg Ala
1               5                   10                  15

gct agg aag aaa ttt cct cct cct tca ttt tac atg cct ctt ttg gtt     97
Ala Arg Lys Lys Phe Pro Pro Pro Ser Phe Tyr Met Pro Leu Leu Val
            20                  25                  30

agt tct gat aag gca cca tat agg gtc att ccc agg aat ctt gtc cct     145
Ser Ser Asp Lys Ala Pro Tyr Arg Val Ile Pro Arg Asn Leu Val Pro
        35                  40                  45

att ggt aag ggt aat aaa gat gag cag att ggt tat tgg aat gtt caa     193
Ile Gly Lys Gly Asn Lys Asp Glu Gln Ile Gly Tyr Trp Asn Val Gln
    50                  55                  60

gag cgt tgg cgt at                                                  207
Glu Arg Trp Arg
65

<210> 16
<211> 68
<212> PRT
<213> Human coronavirus
<223> Description of Combined DNA/RNA Molecule: part of
      replicase polyprotein lab

<400> 16
Ser Lys Leu Asn Lys Met Ala Ser Val Asn Trp Ala Asp Asp Arg Ala
1               5                   10                  15

Ala Arg Lys Lys Phe Pro Pro Pro Ser Phe Tyr Met Pro Leu Leu Val
            20                  25                  30

Ser Ser Asp Lys Ala Pro Tyr Arg Val Ile Pro Arg Asn Leu Val Pro
        35                  40                  45

Ile Gly Lys Gly Asn Lys Asp Glu Gln Ile Gly Tyr Trp Asn Val Gln
    50                  55                  60

Glu Arg Trp Arg
65

<210> 17
<211> 207
<212> DNA
<213> Human coronavirus

<220>
<223> Description of Combined DNA/RNA Molecule: part of
      replicase polyprotein lab

<220>
<221> CDS
<222> (3)..(206)

<400> 17
ac aaa aat ttg aat gag ggt gtt ctt gaa tct ttt tct gtt aca ctt     47
   Lys Asn Leu Asn Glu Gly Val Leu Glu Ser Phe Ser Val Thr Leu

1                 5                   10                  15

ctt gat aat caa gaa gat aag ttt tgg tgt gaa gat ttt tat gct agt    95
Leu Asp Asn Gln Glu Asp Lys Phe Trp Cys Glu Asp Phe Tyr Ala Ser
                20                  25                  30

atg tat gaa aat tct aca ata ttg caa gct gct ggt tta tgt gtt gtt    143
Met Tyr Glu Asn Ser Thr Ile Leu Gln Ala Ala Gly Leu Cys Val Val
            35                  40                  45

tgt ggt tca caa act gta ctt cgt tgt ggt gat tgt ctg cgt aag cct    191
Cys Gly Ser Gln Thr Val Leu Arg Cys Gly Asp Cys Leu Arg Lys Pro
        50                  55                  60

atg ttg tgc act aaa t                                              207
Met Leu Cys Thr Lys
    65

<210> 18
<211> 68
<212> PRT
<213> Human coronavirus
<223> Description of Combined DNA/RNA Molecule: part of
      replicase polyprotein 1ab

<400> 18
Lys Asn Leu Asn Glu Gly Val Leu Glu Ser Phe Ser Val Thr Leu Leu
1               5                   10                  15

Asp Asn Gln Glu Asp Lys Phe Trp Cys Glu Asp Phe Tyr Ala Ser Met
            20                  25                  30

Tyr Glu Asn Ser Thr Ile Leu Gln Ala Ala Gly Leu Cys Val Val Cys
        35                  40                  45

Gly Ser Gln Thr Val Leu Arg Cys Gly Asp Cys Leu Arg Lys Pro Met
    50                  55                  60

Leu Cys Thr Lys
65

<210> 19
<211> 71
<212> DNA
<213> Human coronavirus

<220>
<223> Description of Combined DNA/RNA Molecule: part of
      nucleocapsid protein

<220>
<221> CDS
<222> (1)..(69)

<400> 19
agg ggg caa cgt gtt gat ttg cct cct aaa gtt cat ttt tat tac cta    48
Arg Gly Gln Arg Val Asp Leu Pro Pro Lys Val His Phe Tyr Tyr Leu
1               5                   10                  15

ggt act gga cct cat aag gac ct                                    71
Gly Thr Gly Pro His Lys Asp
            20

<210> 20
<211> 23
<212> PRT
<213> Human coronavirus
<223> Description of Combined DNA/RNA Molecule: part of
      nucleocapsid protein

<400> 20
Arg Gly Gln Arg Val Asp Leu Pro Pro Lys Val His Phe Tyr Tyr Leu
 1               5                  10                  15

Gly Thr Gly Pro His Lys Asp
            20

<210> 21
<211> 107
<212> DNA
<213> Human coronavirus

<220>
<223> Description of Combined DNA/RNA Molecule: part of
      replicase polyprotein 1ab

<220>
<221> CDS
<222> (2)..(106)

<400> 21
t agt agt tgt gtt act cgt tgt aat ata ggt ggt gct gtt tgt tca aaa 49
  Ser Ser Cys Val Thr Arg Cys Asn Ile Gly Gly Ala Val Cys Ser Lys
   1               5                  10                  15

cat gca aat ttg tat caa aaa tac gtt gag gca tat aat aca ttt aca   97
His Ala Asn Leu Tyr Gln Lys Tyr Val Glu Ala Tyr Asn Thr Phe Thr
            20                  25                  30

cag gca ggt t                                                     107
Gln Ala Gly
        35

<210> 22
<211> 35
<212> PRT
<213> Human coronavirus
<223> Description of Combined DNA/RNA Molecule: part of
      replicase polyprotein 1ab

<400> 22
Ser Ser Cys Val Thr Arg Cys Asn Ile Gly Gly Ala Val Cys Ser Lys
 1               5                  10                  15

His Ala Asn Leu Tyr Gln Lys Tyr Val Glu Ala Tyr Asn Thr Phe Thr

20                  25                  30

Gln Ala Gly
        35

<210> 23
<211> 10
<212> DNA
<213> Artificial Sequence

<220>
<223> Description of Artificial Sequence: primer
      repSZ-RT

<220>
<221> misc_feature
<222> (1)..(10)

<400> 23
ccactataac                                                            10

<210> 24
<211> 20
<212> DNA
<213> Artificial Sequence

<220>
<223> Description of Artificial Sequence: primer repSZ-1

<220>
<221> misc_feature
<222> (1)..(20)

<400> 24
gtgatgcata tgctaatttg                                                 20

<210> 25
<211> 20
<212> DNA
<213> Artificial Sequence

<220>
<223> Description of Artificial Sequence: primer repSZ-2

<220>
<221> misc_feature
<222> (1)..(20)

<400> 25
ttggtaaaca aaagataact                                                 20

<210> 26
<211> 20
<212> DNA
<213> Artificial Sequence

<220>
<223> Description of Artificial Sequence: primer repSZ-3

<220>
<221> misc_feature
<222> (1)..(20)

<400> 26
ctcttgcagg tataatccta                                                    20


<210> 27
<211> 20
<212> DNA
<213> Artificial Sequence

<220>
<223> Description of Artificial Sequence: primer repSZ-4

<220>
<221> misc_feature
<222> (1)..(20)

<400> 27
tcaatgctat aaacagtcat                                                    20

## Description

The invention relates to the fields of virology and medicine. More in particular the invention relates to the identification of a new coronavirus and to means and methods associated with a virus such as means and methods for typing the virus in various samples and diagnosing of disease, means and methods for developing vaccines and medicaments for the treatment of infected subjects or of subjects at risk thereof.

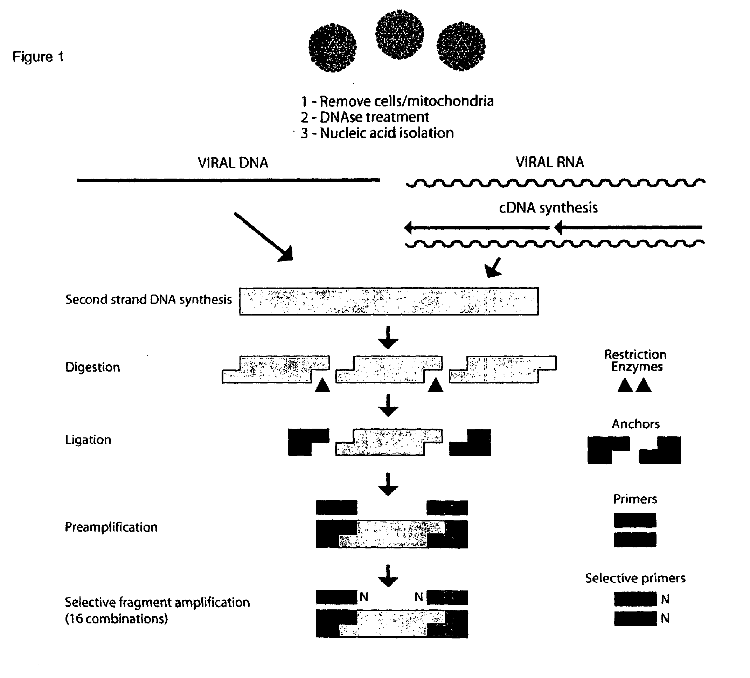

To date there is a range of human diseases with unknown etiology. For many of these a viral origin has been suggested, emphasizing the importance of a continuous search for new viruses^{22, 23, 24}. Major difficulties are encountered when searching for new viruses. First, some viruses do not replicate in vitro, at least not in the cells that are commonly used in viral diagnostics. Second, for those viruses that do replicate in vitro and that cause a cytopathic effect (CPE), the subsequent virus-identification methods may fail. Antibodies raised against known viruses may not recognize the cultured virus and virus specific PCR methods may not amplify the new viral genome. We have developed a method for virus discovery based on the cDNA amplified restriction fragment length polymorphism technique (cDNA-AFLP). With this technique, RNA or DNA is reproducibly amplified. There is no need to have prior knowledge of the sequence of the target gene¹. Generally the cDNA-AFLP method is used to monitor differential gene expression, however, we modified this method such that it can amplify viral sequences either directly from patient blood-plasma/serum samples or indirectly from CPE-positive virus culture (Figure 1). In the modified Virus-Discovery-cDNA-AFLP (VIDISCA) method the mRNA isolation step prior to amplification is replaced by a treatment to selectively enrich for viral nucleic acid. Of relevance to the purification is a centrifugation step to remove residual cells and mitochondria. In addition, a DNAse treatment can be used to remove interfering chromosomal and mitochondrial DNA from degraded cells whereas viral nucleic acid is protected within the viral particle. Finally, by choosing frequently cutting restriction enzymes, the method can be fine-tuned such that most viruses will be amplified.

In January 2003 a 7-month-old child appeared in the hospital with coryza, conjunctivitis and fever. Chest radiography showed typical features of bronchiolitis and a nasopharyngeal aspirate specimen was collected (sample nr: NL63) five days after the onset of disease. All diagnostic tests on this sample for respiratory syncytial virus (RSV), adenovirus, influenza A and B virus, parainfluenza virus type 1, 2 and 3, rhinovirus, enterovirus, HCoV-229E and HCoV-OC43 were negative. Immunofluorescent assays to detect RSV, adenovirus, influenza A and B virus, and parainfluenza virus type 1, 2 and 3 in cultures of the virus remained negative. Acid lability and chloroform sensitivity tests demonstrated that the virus was most likely enveloped and not a member of the Picornavirus group.

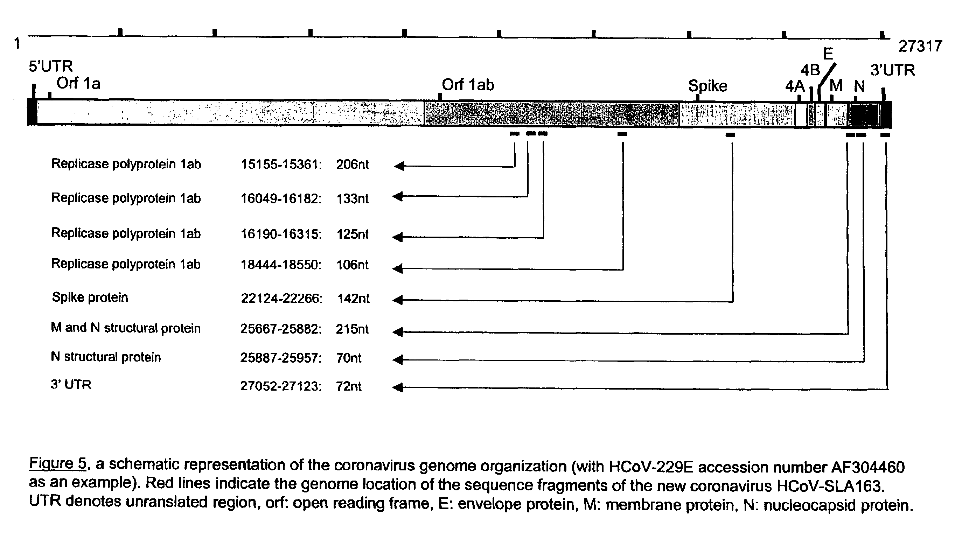

VIDISCA analysis and subsequent sequencing of cloned fragments revealed that the child was infected by a previously unknown member of the Coronavirus family. Coronaviruses are characterized by a very long non-segmented, single-stranded, (+) sense RNA of approximately 27-31 kb. This is the longest genome of any known RNA virus. The genome has a 5' methylated cap and 3' poly-A and functions directly as mRNA.
The newly found coronavirus, (designated HCoV-NL63) was characterized and several of the isolated fragments were sequenced.. The sequences of a number of the fragments are depicted in table 3. The location of the fragments in the large genomic RNA is depicted in figure 5. The invention therefore, in one aspect, provides an isolated or recombinant virus comprising a nucleic acid sequence as depicted in table 3, or a functional part, derivative or analogue of said virus. With the aid of the identifying prototype fragments it is possible to further sequence the genome. One way of doing this by primer walking on the genome. A primer is directed to a region of which the sequence is known and this primer is used to sequence a flanking region that is as yet unknown. A subsequent primer can be generated against the newly identified sequence and a further region can be sequenced. This procedure can be repeated until the entire sequence of the virus is elucidated. As a source of the virus one may turn Dr. C van der Hoek, Department of Human Retrovirology, Academic Medical Center, University of Amsterdam, Amsterdam, The Netherlands

Alignments of the determined nucleic acid sequences revealed the reading frame used in the sequences found, accordingly the invention further provides an isolated or recombinant virus comprising an amino acid sequence as depicted in (table 3). or a functional part, derivative or analogue of said virus. A particular amino acid sequence can be produced from a variety of nucleic acids depending on the codons used. Thus the invention further provides a nucleic acid encoding an amino acid sequence as depicted in (table 3). Further provided is an isolated or recombinant virus comprising a nucleic acid sequence encoding an amino acid sequence as depicted in (table 3), or a functional part, derivative or analogue of said virus.

Coronaviruses as many other types of viruses acquire a plurality of spontaneous and selected mutations upon spreading of the virus through the subject population and/or during culturing ex vivo. Moreover, artificial mutations having no recognized counterpart in nature can be introduced into the sequence of the prototype virus or a derivative thereof, without altering the viral- and/or disease causing properties of the virus. Having characterized the prototype of the newly discovered subtype gives access to this group of viruses belonging to the same subtype. Thus the invention further provides an isolated or recombinant virus comprising a nucleic acid sequence that is approximately 80% homologous to a sequence as depicted in table 3, or 80 % homologous to an amino acid sequence depicted in Table 3 (. Preferably the homology is at least 90%, more preferably at least 95% and even more preferably at least 99%.

The respective prototype fragments were compared with a database of viral sequences and hits having a particularly high homology are mentioned in the tables 5 and 6. It may be noted that the compared fragments do not share extensive homology with any of the currently known Coronaviruses. The invention thus provides an isolated and/or recombinant virus comprising an amino acid sequence which is more than 89% homologous to 163- 2 amino acid sequence as depicted in Table 3. Preferably said homology is at least 90%, more preferably at least 95% and even more preferably at least 99%.
Further provided is an isolated or recombinant virus comprising an amino acid sequence which is more than 60 % homologous to 163- 4 amino acid sequence as depicted in Table 3. Preferably said homology is at least 90%, more preferably at least 95% and even more preferably at least 99%.
Further provided is an isolated or recombinant virus comprising a nucleic acid sequence which is more than 85 % homologous to 163- 9 nucleic acid sequence as depicted in Table 3. Preferably said homology is at least 90%, more preferably at least 95% and even more preferably at least 99%.
Further provided is an isolated or recombinant virus comprising an amino acid sequence which is more than 94 % homologous to 163- 10 amino acid sequence as depicted in Table 3. Preferably said homology is at least 90%, more preferably at least 95% and even more preferably at least 99%.
Further provided is an isolated or recombinant virus comprising an amino acid sequence which is more than 50 % homologous to 163- 11 amino acid sequence as depicted in Table 3. Preferably said homology is at least 90%, more preferably at least 95% and even more preferably at least 99%.
Further provided is an isolated or recombinant virus comprising an amino acid sequence which is more than 87 % homologous to 163- 14 amino acid sequence as depicted in Table 3. Preferably said homology is at least 90%, more preferably at least 95% and even more preferably at least 99%.
Further provided is an isolated or recombinant virus comprising an amino acid sequence which is more than 83 % homologous to 163- 15 amino acid sequence as depicted in Table 3. Preferably said homology is at least 90%, more preferably at least 95% and even more preferably at least 99%.
Further provided is an isolated or recombinant virus comprising an amino acid sequence which is more than 78 % homologous to 163- 18 amino acid sequence as depicted in Table 3. Preferably said homology is at least 90%, more preferably at least 95% and even more preferably at least 99%.
Further provided is an isolated or recombinant virus comprising a nucleic acid sequence which is at least 50 % homologous to a nucleic acid sequence as depicted in Table 3. Preferably said homology is at least 80%, more preferably at least 90%, more preferably at least 95% and even more preferably at least 99%.

The invention also provides a functional part, derivative and/or analogue of an isolated and/or recombinant HCoV-NL63 virus. A part of a virus can be a membrane containing part, a nucleocapsid containing part, a proteinaceous fragment and/or a nucleic acid containing part. The functionality of the part varies with the application chosen for the part, for instance, part of the virus may be used for immunization purposes. In this embodiment the functionality comprises similar immunogenic properties in kind as the entire virus not necessarily in amount. Another use of the virus is the infectivity of the virus, for instance, for in vitro (or in vivo) culture, in this embodiment the functionality comprises a similar infectivity in kind not necessarily in amount. Many other functionalities may be defined, as there are many different uses for viruses, non-limiting examples are the generation of chimeric viruses, (i.e. with one or more other (corona) viruses, and the generation of viral vectors for vaccination and/or gene therapeutic purposes.

Such viruses and/or vectors also contain a functional part of HCoV-NL63 and are thus also encompassed in the present invention. A functional derivative of a virus of the invention is defined as a virus that has been altered such that the properties of said compound are essentially the same in kind, not necessarily in amount. A derivative can be provided in many ways, for instance through nucleotide substitution (preferably "wobble" based), through (conservative) amino acid substitution, subsequent modification, etcetera.
Analogous compounds of a virus can also be generated using methods in the art. For instance, a chimeric virus can be produced, or an HCoV-NL63 virus having a chimeric protein. For instance, HCoV-NL63 can be rendered more immunogenic by generating a cell surface associated fusion protein comprising at least part of an HCoV-NL63 surface protein and a non-HCoV-NL63 immunogenic part. HCoV-NL63 virus comprising such chimeric protein can be used for inducing an enhanced immune response in a host, for instance for vaccination purposes.
As used herein, the term "a virus of the invention" is meant to also comprise a functional part, derivative and/or analogue of said virus.

The three groups of coronaviruses are associated with a variety of diseases of humans and domestic animals, including gastroenteritis and upper and lower respiratory tract disease. The human coronaviruses HCoV-229E and HCoV-OC43 are associated with mild disease (the common cold) but more severe disease is observed in children ¹⁶, albeit at a very low incidence. Several coronaviruses cause a severe disease in animals and SARS-CoV is the first example of a coronavirus that causes severe disease in humans. However, it should be emphasized that a substantial part of respiratory disease cases in humans remains undiagnosed. For instance, a recent survey of respiratory viruses in hospitalized children with bronchiolitis in Canada could not reveal a viral pathogen in about 20% of the patients¹⁷. The fact that we identified the new coronavirus in a child with bronchiolitis shows that HCoV-NL63 is a pathogenic respiratory virus.
When considering that the HCoV-NL63 is a pathogenic respiratory virus able to cause bronchiolitis in infected children, the interesting question remains why HCoV-NL63 was not recognized previously by cell culture. We found that the virus can be cultured in monkey kidney cells (tMK or LLC-MK2 cells), cells that are often used in a routine diagnostic setting and one might therefore speculate that HCoV-NL63, like SARS-CoV, was newly introduced from an animal reservoir into the human population or that this is a human virus that recently broadened its host cell range. Clearly it is of importance to study the prevalence of HCoV-NL63 infection, and screening specimens from patients with respiratory tract disease using the HCoV-NL63 diagnostic RT-PCR will shed light on this issue.
It is remarkable that the new human coronavirus was harvested from tMK cells and LLC-MK2 cells since coronaviruses are typically fastidious in cell culture with a narrow host range. However, both SARS-CoV and HCoV-NL63 seem to replicate efficiently in monkey kidney cells (Vero-E6 cells and NCI-H292 cells for SARS-CoV). The recently described genome of SARS-CoV has several exclusive features, including some unique open reading frames that are probably of biological significance^{15,18}. We will therefore analyze the complete genome sequence of HCoV-NL63 to screen for similarities and differences with SARS-CoV that may determine the expanded host cell range and enhanced pathogenicity of these viruses.

HCoV-NL63 is associated with a particular phenotype in infected subjects. The phenotype can encompass bronchiolitis, coryza, conjunctivitis and fever and may further encompass other respiratory problems and diarrhea. In one embodiment the invention thus further provides an isolated and or recombinant virus of the invention (having one or more of the above mentioned homology) wherein said virus or functional part, derivative and/or analogue further comprises the capability to induce an HCoV-NL63 related disease or symptom in a subject. In another embodiment the invention provides an isolated and/or recombinant virus of the invention further comprising the property to cause CPE in tertiary monkey kidney cells (tMK; Cynomolgus monkey³⁷) and/or upon passage onto the monkey cell line LLC-MK2 (ECCAC 85062804, ATCC CCL-7). In a preferred embodiment said virus does not produce CPE in Vero- cells **(ATCC CRL-1586)**^{**34**}**.**

The invention further provides a nucleic acid as depicted in table 3, and an amino acid sequence as depicted in Table 3, or a functional part and/or equivalent of such a nucleic acid and/or amino acid sequence. A functional equivalent of said nucleic acid comprises the same hybridization properties in kind, not necessarily in amount, as said nucleic acid (or part thereof). A functional equivalent of an amino acid sequence of the invention comprises the same immunogenic properties in kind, not necessarily in amount, as said amino acid sequence (or part thereof). A part of a nucleic acid of the invention comprises at least 15 nucleotides, preferably at least 20, more preferably at least 30 nucleotides. A part of an amino acid sequence comprises at least 5 amino acids in peptidic linkage with each other, more preferably at least 8, and more preferably at least 12, more preferably at least 16 amino acids. In a preferred embodiment said nucleotides and/or amino acids are at least semi-consecutive, more preferably, said nucleotides and/or amino acids are consecutive. An equivalent of a nucleic acid and/or amino acid sequence of the invention or part thereof comprises at least 80% homology to a nucleic acid and/or amino acid sequence of the invention, preferably at least 90% homology, more preferably at least 95% and even more preferably at least 99% homology to a nucleic acid and/or amino acid sequence of the invention or a part thereof.

The invention further provides a primer and/or probe, capable of specifically hybridizing to a nucleic acid of a virus or functional part, derivative or analogue according to the invention, preferably a primer and/or probe, capable of specifically hybridizing to a nucleic acid sequence as depicted in Table 3. More preferably, a primer and/or probe, which is capable of hybridizing to said nucleic acid under stringent conditions. In a particular preferred embodiment is provided a primer and/or probe, comprising a sequence as depicted in Table 7.

The art knows many ways in which a specific binding member can be generated against an identified nucleic acid, lipid and/or amino acid sequence. Such specific binding members may be of any nature but are typically of a nucleic acid and/or proteinaceous nature. The invention thus further provides an isolated molecule capable of specifically binding a virus, nucleic acid and/or amino acid or functional part, derivative or analogue thereof according to the invention. Said isolated molecule is also referred to as specific binding member. Preferably said specific binding member is capable of specifically binding at least part of a nucleic acid sequence as depicted in table 3 and/or at least part of an amino acid sequence as depicted in Table 3. In a preferred embodiment said binding member is a proteinaceous molecule. Preferably an antibody or a functional part, derivative and/or analogue thereof. A specific binding member preferably comprises a significantly better binding property for the HCoV-NL63 virus compared to unrelated control. However, for instance for antibodies, it is possible that the epitope specifically recognized in HCoV-NL63 is also present in a limited number of other molecules. Thus though the binding of the binding member may be specific, it may recognize also other molecules than those present in HCoV-NL63. This cross-reactivity is to be separated from a-specific binding and is a general property of antibodies. Cross-reactivity does not usually hinder the selection of suitable specific binding members for particular purposes. For instance a specific binding member that also recognized a protein in liver cells can be used in many applications even in the presence of liver cells, where additional information such as location in the cell can often be used to discriminate.

One source of an antibody of the invention is the blood of the infected subjects screened for the virus of the present invention. One may further characterize B-cells obtained from said subject. A suitable B-cell may be cultured and the antibody collected. Alternatively, the antibody may be sequenced from this B-cell and generated artificially. Another source of an antibody of the invention can be generated by immuninisation of test animals or using artificial libraries to screen a purified fraction of virus. A functional part of an antibody has essentially the same properties of said antibody in kind, not necessarily in amount. Said functional part is preferably capable of specifically binding an antigen of HCoV-NL63. However, said functional part may bind such antigen to a different extend as compared to said whole antibody. A functional part or derivative of an antibody for instance comprises a FAB fragment or a single chain antibody. An analogue of an antibody for instance comprises a chimeric antibody. As used herein, the term "antibody" is also meant to comprise a functional part, derivative and/or analogue of said antibody.

Once antibody of the invention is obtained, a desired property, such as its binding capacity, can be improved. This can for instance be done by an Ala-scan and/or replacement net mapping method. With these methods, many different proteinaceous molecules are generated, based on an original amino acid sequence but each molecule containing a substitution of at least one amino acid residue. Said amino acid residue may either be replaced by Alanine (Ala-scan) or by any other amino acid residue (replacement net mapping). Each variant is subsequently screened for said desired property. Generated data are used to design an improved proteinaceous molecule.

There are many different ways in which a specific binding member can be generated. In a preferred embodiment the invention provides a method for producing a specific proteinaceous binding member comprising producing proteinaceous molecules capable of binding a virus according to the invention or to a functional part, derivative or analogue, and selecting a proteinaceous molecule that is specific for said virus. If need be, the method may be used to generate a collection of proteinaceous molecules capable of binding to said virus or functional part, derivative and/or analogue thereof and selecting from said collection one or more binding members capable of specifically binding said virus or functional part, derivative and/or analogue thereof.

Any specific binding member is characteristic for the HCoV-NL63virus of the invention. Thus a virus that is specifically reactive with such binding member is an HCoV-NL63 virus and thus provided by the invention. Thus the invention provides an isolated and/or recombinant virus that is immunoreactive with specific binding member of the invention, preferably a proteinaceous binding member. The invention further provides a composition of matter comprising isolated HCoV-NL63 virus, and/or a virus essentially corresponding to HCoV-NL63. The term, a virus "essentially corresponding to HCoV-NL63" refers to HCoV-NL63 viruses which are either identical to the HCoV-NL63 strain described hereinabove, or which comprises one or more mutations compared to the said HCoV-NL63strain. These mutations may include natural mutations or artificial mutations. Said mutations of course should allow detection with a specific binding member of HCoV-NL63, not necessarily with all of the specific binding members). Said mutations should allow the detection of the variants using common detection methods such as antibody interaction, amplification and/or hybridization.

Considering that specific binding members are important molecules for instance for diagnostic purposes, the invention further provides the use of a virus of the invention or functional part, derivative and/or analogue thereof, for detecting a molecule capable of specifically binding said virus in a sample. Further provided is the use of a nucleic acid and/or amino acid sequence of a virus or functional part, derivative or analogue as defined by the invention, for detecting a molecule capable of specifically binding said virus or functional part, derivative and/or analogue in a sample. Preferably said nucleic acid and/or amino acid sequence comprises a sequence as depicted in table 3 or Table 3 or a functional part, derivative or analogue thereof. Preferably said part is at least 30 nucleotides and/or amino acids long wherein said part preferably comprises more than 95% sequence identity, preferably more than 99%. In a preferred aspect said specific binding member comprises a specific ligand and/or antibody of said virus.

Further provided is a primer and/or probe according to the invention, a specific binding member of the invention, and/or a nucleic acid of a virus or functional part, derivative or analogue according to the invention, for detecting and/or identifying a HCoV-NL63 coronavirus or part thereof in a sample. Preferably, said nucleic acid comprises a sequence as depicted in table 3.

HCoV-NL63 virus may be used to generate an immune response in a subject. This can be useful for instance in vaccination strategies. Thus the invention further HCoV-NL63 provides HCoV-NL63 virus or functional part, derivative or analogue thereof for use as a vaccine or medicament. The medicament use is typically when the subject is already infected with the virus and the immunogen is used to augment the immune response against the virus. The invention further provides a specific binding member of the invention for use as a vaccine or medicament. This use is particularly favorable for when the specific binding member comprises a proteinaceous molecule, preferably an antibody or functional part, derivative and/or analogue thereof. Such an antibody can provide passive immunity but may also have active components such as proteases attached to it. The medicament use may again be the case wherein a subject infected with an HCoV-NL63 virus is treated with the specific binding member.

Vaccines may be generated in a variety of ways. One way is to culture the HCoV-NL63 virus for example on the mentioned monkey cell line(s) and to use inactivated virus harvested from the culture. Alternatively, attenuated virus may be used either inactivated or as a live vaccine. Methods for the generation of coronavirus vaccines may be adapted to produce vaccines for the HCoV-NL63 of the invention. The invention thus further provides the use of an HCoV-NL63 virus or functional part, derivative or analogue thereof for the preparation of a vaccine against a coronaviral genus related disease. The invention further provides the use of a specific binding member of the invention for the preparation of a vaccine or medicament against a coronaviral genus related disease. Further provided is the use of an HCoV-NL63 virus or functional part, derivative or analogue thereof, a specific binding member of the invention, a nucleic acid of the invention or a primer and/or probe of the invention for diagnosis of a coronaviral genus related disease. Preferably said coronaviral genus related disease comprises a HCoV-NL63coronavirus related disease.

Further provided is a vaccine comprising an HCoV-NL63 virus or functional part, derivative or analogue thereof and/or a specific binding member of the invention. Also provided is a medicament comprising an HCoV-NL63virus or functional part, derivative or analogue thereof and/or a specific binding member of the invention. Preferably said vaccine or medicament is used for at least in part preventing and/or treating a HCoV-NL63 related disease.

An important use of the present invention is the generation of a diagnostic tool for determining whether a subject is suffering from an HCoV-NL63 virus infection or has been exposed to an HCoV-NL63 virus infection. Many different diagnostic applications can be envisioned. They typically contain an identifying component allowing the typing of the virus that is or was present in the subject. One diagnostic tool for HCoV-NL63 makes use of the particular proliferation characteristics of the virus in various cell lines. It replicates in the mentioned preferred monkey cell lines but does not replicate in Vero- cells. This property can be used to discriminate HCoV-NL63 from other known coronaviruses. Thus in one aspect the invention provides a diagnostic kit comprising at least one of the preferred monkey cell lines, preferably the tertiary monkey kidney cells (tMK; Cynomolgus monkey or the monkey cell line LLC-MK2.
Many modern diagnostic kits comprise a specific binding member (to detect the virus or virus infected cells) and/or an HCoV-NL63 virus or a functional part, derivative and/or analogue thereof and/or amino acid of the invention or a functional part, derivative and/or analogue thereof (for detecting antibodies in blood components of the diagnosed subject). Many other current diagnostic kits rely on identification of HCoV-NL63 virus specific nucleic acid in a sample. There are various ways in which such an assay may be implemented one is a method for detecting an HCoV-NL63 virus or functional part, derivative or analogue thereof in a sample, comprising hybridizing and/or amplifying a nucleic acid of said virus or functional part, derivative or analogue with a primer and/or probe according to the invention and detecting hybridized and/or amplified product. The invention thus also provides a diagnostic kit comprising an HCoV-NL63 virus or functional part, derivative or analogue thereof, a specific binding member according to the invention and/or a primer/probe according to the invention.

Further provided is a method for treating an individual suffering from, or at risk of suffering from, a HCoV-NL63 related disease, comprising administering to said individual a vaccine or medicament according to the invention. Also provided is a method for determining whether an individual suffers from a HCoV-NL63 related disease, comprising obtaining a sample from said individual and detecting a HCoV-NL63 virus or functional part, derivative or analogue thereof in said sample with a method and/or diagnostic kit of the invention.

Further provided is an isolated or recombinant nucleic acid encoding a virus or functional part, derivative and/or analogue according to the invention and a nucleic acid according to the invention, comprising at least a functional part of a sequence as depicted in Table 3. Further provided is an amino acid sequence encoded by a nucleic acid according to the invention, and an amino acid sequence according to the invention, comprising at least a functional part of a sequence as depicted in Table 3. Further provided is a proteinaceous molecule capable of specifically binding HCoV-NL63, obtainable by a method according to the invention and, the use of such a proteinaceous molecule in a vaccine or a diagnostic method for the detection of HCoV-NL63.

### EXAMPLES

### cDNA-AFLP for virus discovery

We modified the cDNA-AFLP technique such that it can amplify viral sequences from blood-plasma/serum samples or from CPE-positive culture supernatants (Figure 1). In the adjusted method the mRNA isolation step prior to amplification is replaced by a treatment to purify viral nucleic acid. Of importance to the purification is a centrifugation step to remove residual cells and mitochondria. In addition, a single DNAse treatment is sufficient to get rid of interfering chromosomal DNA and mitochondrial DNA from broken down cells and finally, by choosing frequent cutting restriction enzymes, the method is fine-tuned such that the majority of viruses will be amplified. With this so-called Virus Discovery cDNA-AFLP (VIDISCA) we were able to amplify viral nucleic acids from EDTA-plasma of a person with hepatitis B virus infection and a person with an acute Parvo B19 infection (results not shown). The technique can also detect HIV-1 in a positive culture supernatant demonstrating its capacity to identify both RNA and DNA viruses (results not shown).

To eliminate residual cells, 110 µl of virus culture supernatant was spun down for 10 min at maximum speed in an Eppendorf microcentrifuge (13500 rpm). One hundred µl was transferred to a fresh tube and DNAse treated for 45 minutes at 37°C using 15 µl of DNAse buffer and 20 Units of DNAse I (Ambion). The DNAse treatment was included to get rid of chromosomal DNA from broken down cells. After this 900 µl of L6 lysis buffer and 40 µl of silica suspension was added and nucleic acids were extracted as described by Boom⁴. The viral nucleic acids were eluted in 40 µl H₂O. With 20 µl eluate the reverse transcription was performed using 2.5 µg random hexamers (Amersham Bioscience), 200 U MMLV-RT (Invitrogen) in a buffer containing 10 mM Tris-HCl pH 8.3, 50 mM KCl, 0.1% Triton X-100, 4.8 mM MgCl2, and 0.4 mM of each dNTP. The sample was incubated at 37°C for 90 minutes. Subsequently the second strand DNA synthesis was performed using 26 U Sequenase II (Amersham Bioscience), 7.5 U RNAse H (Amersham Bioscience) in 0.25 mM dNTPs each, 17.5 mM MgCl2 and 35 mM Tris-HCl pH 7.5. After the incubation at 37°C for 90 minutes a phenol/chloroform extraction was performed followed by an ethanol precipitation. The pellet was dissolved in 30 µl of H₂O. The cDNA-AFLP was performed essentially as described by Bachem¹ with some modifications. The dsDNA was digested with the HinP I and MseI restriction enzymes (New England Biolabs) according to the manufacturers protocol. After the digestion, MseI adaptor and HinP I adaptor (see below) are added together with 5U ligase enzyme (Invitrogen) and ligase buffer, followed by an additional incubation of 2 hrs at 37°C. The MseI adaptor and HinP I adaptor were prepared previously by mixing a top strand oligo for the MSE and the HinP1 adaptors (Table 1) with a bottom strand oligo for the MSE adaptor and for the HinP1 adaptor, incubate at 65° C. followed by cooling down to room temperature in the presence of a 1:40 dilution of ligase buffer.

The first PCR was performed with 10 µl of ligation mixture as input, 2.5 U of AmpliTaq polymerase (Perkin-Elmer), 100 ng of HinPI standard primer and 100 ng of MseI standard primer. The PCR reaction was performed according to the profile 5min 95C; 20 cycles of: 1min 95°C-1min 55°C-2min 72°C; 10 min 72 °C. Five µl of first PCR product was used as input in the second "selective" amplification step containing 100 ng of HinPI-N primer and 100 ng MseI-N (sequence of the standard primers extended with one nucleotide) and 2 U AmpliTaq polymerase. The selective PCRs were amplified according to the profile of the "touch down PCR": 10 cycles of 60 sec 94° C-30 sec 65° C-1 min 72°C over which the annealing temperature was reduced from 65°C with 1 °C with each cycle, followed by 23 cycles: 30 sec 94°C.30 sec 56 °C-1 min 72 °C. Finally the sample was incubated for 10 min at 72°C. The PCR products were evaluated on 4% Metaphor® gels (Cambrex, Rockland, USA). If the bands on the gel were very faint the PCR products were concentrated by vacuum drying using 60 µl of the PCR product. The PCR fragments of interest were cut out of gel and DNA was eluted from the gel using the Qiagen gel purification kit according to the manufacturer's protocol. The PCR products were cloned using pCR® 2.1-TOPO plasmid (Invitrogen) and chemically competent One Shot E. coli (Invitrogen). A PCR on the colony was performed and this PCR product was input for sequencing the insert using Big Dye terminator chemistry (Applied Biosystems). The reverse transcription step was excluded, only HinP I digestion and adaptor ligation was performed, the first PCR was performed with 35 cycles instead of 20 and those first PCR fragments were visualized on agarose gel electrophoresis.

### DNA sequencing and analysis.

Coronavirus-PCR product containing plasmids were sequenced with the BigDyeTM Terminator Cycle Sequencing Kit (Applied Biosystems, Foster City, Calif.), using the -21 M13RP and T7 primers. Electrophoresis of sequencing reaction mixtures was performed with an Applied Biosystems 377 automated sequencer, following the manufacturer's protocols. The Sequence Navigator (version 1.01) and Auto Assembler (version 2.1) software packages (ABI, California, USA) were used to analyze all sequencing data. Sequences were compared to all sequences in the Genbank database using the BLAST tool of the NCBI webpage: http://www.ncbi.nlm.nih.gov/blast. For phylogenetic analysis the sequences were aligned using the ClustalX software package³⁴ with the following settings: Gap opening penalties: 10.00; Gap extension penalty 0.20, Delay divergent sequences switch at 30% and transition weight 0.59. Phylogenetic analysis was carried out using the neighbor-joining method of the MEGA program³⁵. The nucleotide distance matrix was generated either by Kimura's 2-parameter estimation or by the p-distance estimation³⁶. Bootstrap resampling (500 replications) was employed to place approximate confidence limits on individual branches.

### Determining the nucleotide sequence of the complete HCoV-NL63 genome.

Using a combination of specific primers, located in the already sequenced domains of the HCoV-NL63 genome, and the proprietary PALM-method (WO 0151661) we are in the process of cloning and determining the full-length genomic sequence for this new coronavirus. Using a combination of 5'-oligonucleotides located in the analyzed part of the HCoV-NL63 genome and a 3' tagged random primer (JZH2R) additional fragments were amplified using a nested RT-PCR protocol similar to the one mentioned previously.

### Isolation of SZ 163

In January 2003 a 7-month-old child appeared in hospital with coryza, conjunctivitis and fever. Chest radiography showed typical features of bronchiolotis and four days after the onset of disease a nasopharyngeal aspirate specimen was collected (sample nr: HCoV-NL63). All routinely used tests on this sample for adenovirus, respiratory syncytial virus (RSV), influenza A and B, parainfluenza 1, 2 and 3, rhinovirus, HCoV-229E and HCoV-OC43 were negative. The clinical sample was subsequently inoculated onto a variety of cells including human fibroblast lung (HFL) cells, tertiary monkey kidney cells (tMK; Cynomolgus) and R-HeLa cells. A CPE was detected exclusively on tMK cells and first noted at eight days post-inoculation. The CPE was diffuse with a refractive appearance in the affected cells followed by cell detachment after 7 days. More pronounced CPE was observed upon passage onto LLC-MK2 cells. Besides overall cell rounding, moderate cell enlargement was observed. Additional subcultering on human endothelial lung cells, HFL, Rhabdomyosarcoma cells and Vero cells remained negative for CPE. Immunofluorescent assays to detect influenzavirus A and B, RSV, adenoviruses or parainfluenza virus types 1, 2 or 3 in the culture remained negative
The culture supernatant of infected LLC-MK2 cells was subsequently analyzed by VIDISCA. As control we used the supernatant of uninfected LLC-MK2 cells. After the second PCR amplification step, several DNA fragments were present in the test sample but not in the control. These fragments were cloned and sequenced. A Blast search in GenBank revealed that 8 of 16 fragments had sequence similarity to the family of corona viruses with the highest homology the human corona virus 229E (Tables 4 and 5).

Phylogenetic analysis of a 270 nt fragment of the replicase 1B region indicated that we identified a distinct new member of the coronavirus group 1. With the VIDISCA technique, 8 HCOV-163-specific fragments, named 163-2, 163-4, 163-9, 163-10, 163-11, 163-14, 163-15 and 163-18 were isolated, cloned, sequenced and aligned with the relevant sequences from GenBank. The Genbank accession number of the used sequences are: MHV (mouse hepatitis virus): AF201929; HCoV-229E: AF304460; PEDV (porcine epidemic diarrhea virus): AF353511; TGEV (transmissible gastroenteritis virus): AJ271965; SARS-CoV: AY278554; IBV (avian infectious bronchitis virus): NC_001451; BCoV (bovine coronavirus): NC_003045; FCoV (feline coronavirus): Y13921 and X80799; CCoV (canine coronavirus): AB105373 and A22732; PRCoV (porcine respiratory coronavirus): M94097; FIPV (feline infectious peritonitis virus): D32044. Position of the HCoV-NL63 fragments compared to HCoV-229E (AF304460): Replicase 1AB gene: 15155-15361, 16049-16182, 16190-16315, 18444-18550, Spike gene: 22124-22266; Nucleocapsid gene: 25667-25882 and 25887-25957; 3'UTR: 27052-27123. Branch lengths indicate the number of substitutions per sequence. From the most closely related species sequence identity scores were calculated (Tables 5 and 6).

Also the deduced amino acid sequence were aligned to the corresponding domains in the open reading frames of related corona (-like) viruses (Table 6).

The human corona viruses account for 10 to 30% of the common colds in man⁷, and it is not unusual to find a coronavirus in a child with a respiratory illness. However, it is striking that the virus HCoV-NL63 was harvested from LLC-MK cells. Human Corona virus 229E and OC-43 are known for there inability to replicate on monkey kidney cells. Intriguingly, the newly identified human corona virus that is responsible for SARS is also able to replicate in monkey kidney cells 30.

### Propagation of HCoV-NL63 in cell culture

A nasopharyngeal aspirate was collected 4 days after the onset of symptoms. The specimen was tested for the presence of adenovirus, RSV, influenza A, influenza B, and parainfluenza type 1, 2 an 3 using the Virus Respiratory Kit (Bartels: Trinity Biotech plc, Wicklow Ireland). In addition, PCR diagnosis for rhinoviruses, meta-pneumovirus and HCoV-OC43 and HCoV-229E were performed^{2, 10}. The original nasopharyngeal aspirate was subsequently inoculated onto a variety of cell cultures including HFL cells, tMK cells and R-HeLa cells. The tubes were kept in a roller drum at 34°C and observed every 3 to 4 days. Maintenance medium was replenished every 3 to 4 days. Two different types of medium were implemented: Optimem 1 (Gibco) without bovine fetal serum was used for the tMK cells and MEM Hanks' /Earle's medium (Gibco) with 3% bovine fetal serum was used for the remaining cell types. On the virus culture direct staining was performed with pools of fluorescent-labeled mouse antibodies against influenzavirus A and B, RSV and adenoviruses (Imagen, DAKO). Indirect staining was performed for parainfluenza virus types 1, 2 or 3 with mouse antibodies (Chemicon, Brunschwig, Amsterdam Netherlands) and subsequent staining with labeled rabbit anti-mouse antibodies (Imagen, DAKO).

### Method to detect HCoV-NL63 in nasopharyngeal swabs.

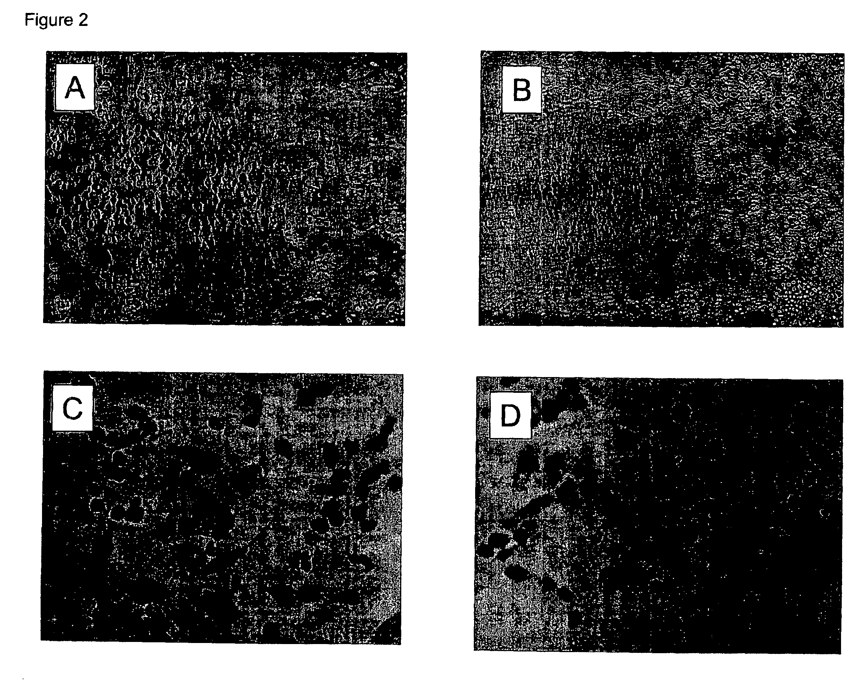

For the diagnostic RT-PCR, nucleic acids were extracted by the Boom method⁴ 4 from 50 µl virus supernatant or 50 µl suspended nasopharyngeal swab. The reverse transcription was performed as described above with the exception that 10 ng of reverse transcription primer repSZ-RT (Table 7) was used. The entire RT mixture was added to the first PCR mixture containing 100 ng of primer repSZ-1 and 100 ng of primer repSZ-3. The PCR reaction was performed according to the profile 5 min 95 °C; 20 cycles of: 1 min 95°C - 1min 55°C - 2 min 72°C; 10 min 72°C. A nested PCR was started using 5 µl of the first PCR with 100 ng of primer repSZ-2 and 100 ng of primer repSZ-4.
Twenty-five PCR cycles were performed of the same profile as the first PCR. Ten µl of the first and 10 µl of the nested PCR was analyzed by agarose gel electrophoresis (Fig. 2). Cloning and sequencing of the fragments was performed essentially as described above.

### Method of raising polyclonal antibodies

Appropriate domains within the HCoV-NL63 surface proteins (e.g. S-glycoprotein or HE- glycoprotein) can be selected and amplified with suitable oligonucleotides and RT-PCR. The corresponding purified viral antigens can be obtained by expression in a suitable host *(e.g. Yarrowia lipolytica* as previously described³⁸). Female NZW rabbits (approx 4 kg) are primed with 0.5 to 5.0 mg of viral protein antigen preparation. The antigen is suspended in 0.5 ml. of phosphate buffered saline (pH 7.3) and emulsified in an equal volume of complete Freund's adjuvant (CFA). Freund's Adjuvant is a well-established adjuvant system that is appropriate for use in these experiments where small amounts of antigen are used, and where immunogenicity of the antigen (although likely) is unknown. Published guidelines for use will be followed, including limiting injection to 0.1 ml at each site, using CFA only for initial immunization dose. This antigen preparation (1 ml total volume) is injected subdermally in the loose skin on the backside of the rabbit's neck. This injection route is immunologically effective and minimizes the possibility of local inflammation associated with unilateral or bilateral flank injection (such ensuing flank inflammation can impair animal mobility). After resting for 3 weeks, one ml of blood will be removed from the ear artery for a test bleed. Antibodies will be boosted if titers of the desirable antibodies are judged to be too low. Rabbits with adequate antibody levels will be boosted subdermally 1.0 mg of antigen contained in CFA. Boosted animals will be bled after two weeks; i.e., 15 ml of blood will be taken from the ear artery using a heat lamp to dilate the blood vessel. The rabbit will be placed in a commercial restraint, tranquillized with xylazine not more than seven times in total after which the rabbit will be exsanguinated by cardiac puncture following anesthesia using xylazinelketamine.

### Method for Vaccine production

For the production of a subunit vaccine the S-glycoprotein perhaps combined with the HE, M and N proteins, could be expressed in a suitable eukaryotic host (e.g. *Y. lipolytica* or LLC-MK2 cells) and purified using preferentially two small affinity tags (e.g. His-tag or the StrepII tag). After appropriate purification, the resulting viral proteins can be used as a subunit vaccine.

Alternatively the HCoV-NL63 virus can be propagated in a suitable cell line as described above and subsequently treated as described by Wu ¹¹. Briefly the virus is precipitated from culture medium with 20% polyethylene glycol 6000 and purified by ultracentrifugation at 80.000 × g for 4 hours through a discontinuous 40-65% sucrose gradient followed by a linear 5 to 40 % CsCl gradient for 4 hours at 120.000 × g. The resulting virus preparation can be inactivated by heating for 30 minutes at 65° C as described by Blondel³.

*Analysis of S glycoprotein* or any *of the HCOV-NL63 viral proteins binding to an immobilized ligand (e.g. antibody) in* an *optical biosensor.*
Binding reactions were carried out in an IAsys two-channel resonant mirror biosensor at 20°C (Affinity Sensors, Saxon Hill, Cambridge, United Kingdom) with minor modifications. Planar biotin surfaces, with which a signal of 600 arc s corresponds to 1 ng of bound protein/mm2, were derivatized with streptavidin according to the manufacturer's instructions. Controls showed that the viral proteins did not bind to streptavidin-derivatized biotin surfaces (result not shown). Biotinylated antibody was immobilized on planar streptavidin-derivatized surfaces, which were then washed with PBS. The distribution of the immobilized ligand and of the bound S-glycoprotein on the surface of the biosensor cuvette was inspected by the resonance scan, which showed that at all times these molecules were distributed uniformly on the sensor surface and therefore were not micro-aggregated. Binding assays were conducted in a final volume of 30 µl of PBS at 20 ± 0.1°C. The ligate was added at a known concentration in 1 µl to 5 µl of PBS to the cuvette to give a final concentration of S-glycoprotein ranging from 14 to 70 nM. To remove residual bound ligate after the dissociation phase, and thus regenerate the immobilized ligand, the cuvette was washed three times with 50 µl of 2 M NaCl-10 mM Na2HPO4, pH 7.2, and three times with 50 µl of 20 mM HCl. Data were pooled from experiments carried out with different amounts of immobilized antibody (0.2, 0.6, and 1.2 ng/mm2). For the calculation of kₒₙ, low concentrations of ligate (S-glycoprotein) were used, whereas for the measurement of k_{off}, higher concentrations of ligate were employed (1 µM) to avoid any rebinding artefacts. The binding parameters kₒₙ and k_{off} were calculated from the association and dissociation phases of the binding reactions, respectively, using the non-linear curve-fitting FastFit software (Affinity Sensors) provided with the instrument. The dissociation constant (K_{d}) was calculated from the association and dissociation rate constants and from the extent of binding observed near equilibrium.

### Tables

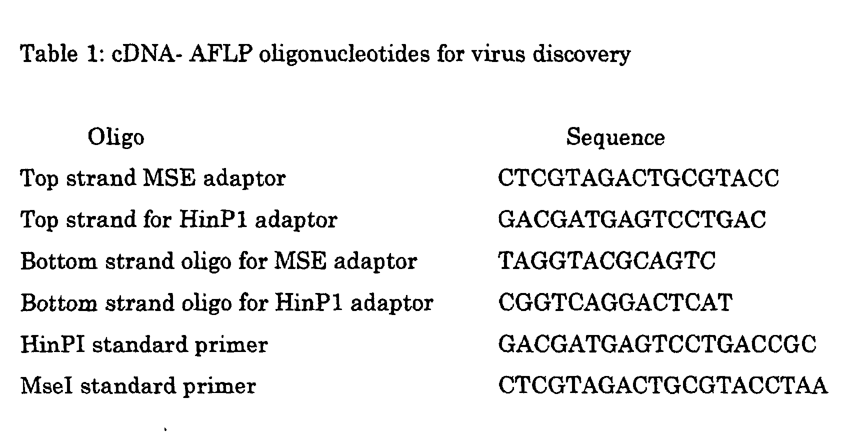

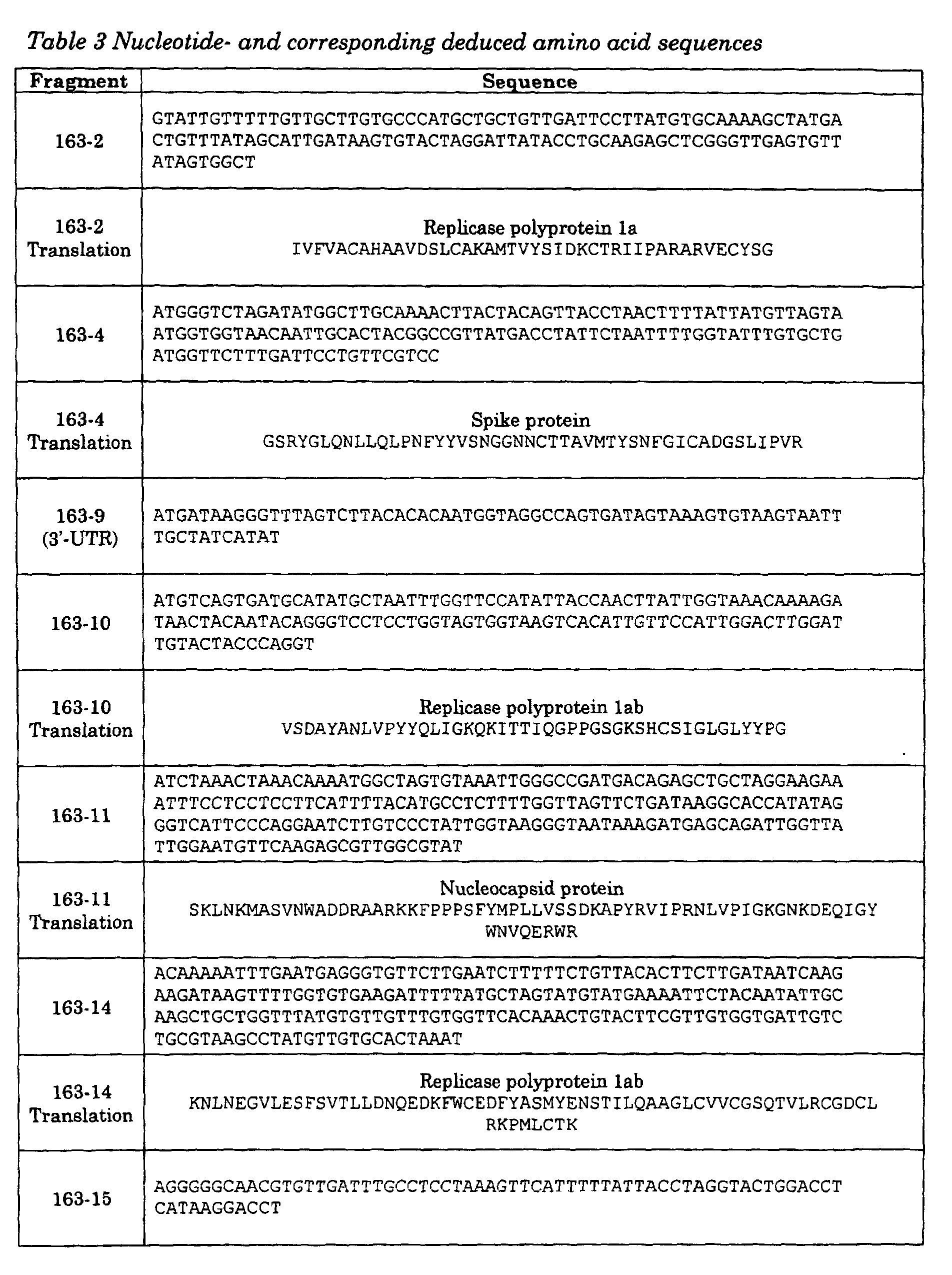

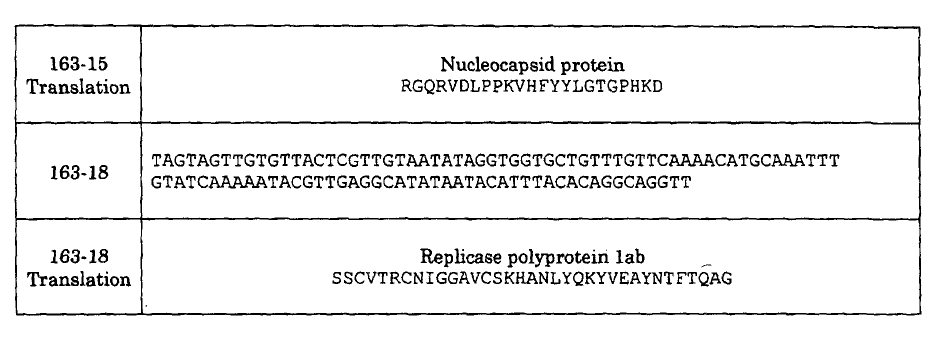

Table 2: Oligonucleotide for PALM extension of the HCOV-NL63 Sequence

Oligonucleotide name, | Application, | Sequence 5'- 3'
JZH2R | 1st PCR | GCTATCATCACAATGGACNNNNNG

**Table 4**

| Identification of cDNA-AFLP fragments | |
|---|---|
| Fragment | Identification best Blast hit |
| 163-2 | replicase polyprotein 1ab [Human coronavirus 229E] |
| 163-4 | spike protein [Human coronavirus 229E] |
| 163-9 | 3'UTR Human coronavirus 229E |
| 163-10 | replicase polyprotein 1ab [Human coronavirus 229E] |
| 163-11 | replicase polyprotein 1ab [Human coronavirus 229E] |
| 163- 14 | replicase polyprotein 1ab [Human coronavirus 229E] |
| 163-15 | nucleocapsid protein [Human coronavirus 229E] |
| 163-18 | replicase polyprotein 1ab [Human coronavirus 229E] |

**Table 5:**

| Pairwise nucleotide sequence homologies between the virus of the present invention and different corona (like) viruses in percentages sequence identity (%) | | | | | | | |
|---|---|---|---|---|---|---|---|
| Fragment | BcoV | MHV | HcoV | PEDV | TGE | SARS | IBV |
| Replicase 1AB 163-2 | 59.6 | 61.2 | 76.7 | 70.5 | 64.3 | 65.8 | 64.3 |
| Spike gene 163-4 | 31.7 | 26.5 | 64.6 | 48.9 | 45.4 | 33.7 | 25.9 |
| 3'UTR 163-9 | 29.5 | 34 | 81.9 | 53.6 | 50 | 31.5 | 38 |
| Replicase 1AB 163-10 | 55.2 | 57.4 | 82 | 73.8 | 69.4 | 64.1 | 65.1 |
| Nucleocapsid 163-11 | 25.5 | 23.8 | 54.9 | 51.5 | 44.6 | 23.3 | 27.6 |
| Replicase 1AB 163-14 | 52.1 | 52.1 | 78.7 | 72.9 | 76.3 | 52.6 | 58.4 |
| Nucleocapsid 163-15 | 29.5 | 35.2 | 71.8 | 63.3 | 60.5 | 25.3 | 45 |
| Replicase 1AB 163-18 | 67.2 | 65.4 | 72.8 | 65.4 | 61.6 | 68.2 | 57 |

**Table 6:**

| Pairwise deduced amino acid sequence homologies between different corona (like) viruses in percentages sequence identity (%) | | | | | | | |
|---|---|---|---|---|---|---|---|
| Fragment | BCoV | MHV | HcoV | PEDV | TGE | SARS | IBV |
| Replicase 1AB 163-2 | 55.8 | 53.4 | 88.3 | 79 | 60.4 | 67.4 | 55.8 |
| Spike gene 163-4 | ND | ND | 56.2 | ND | ND | ND | ND |
| Replicase 1AB 163-10 | 51.1 | 53.3 | 93.3 | 86.6 | 80 | 57.7 | 55.5 |
| Nucleocapsid 163-11 | ND | ND | 48.4 | ND | ND | ND | ND |
| Replicase 1AB 163-14 | 50.7 | 50.7 | 86.9 | 78.2 | 78.2 | 46.3 | 47.8 |
| Nucleocapsid 163-15 | ND | ND | 82.6 | ND | ND | ND | ND |
| Nucleocapsid 163-18 | 63.8 | 63.8 | 77.7 | 69.4 | 69.4 | 58.3 | 55.5 |
| ND = Not Determined | | | | | | | |

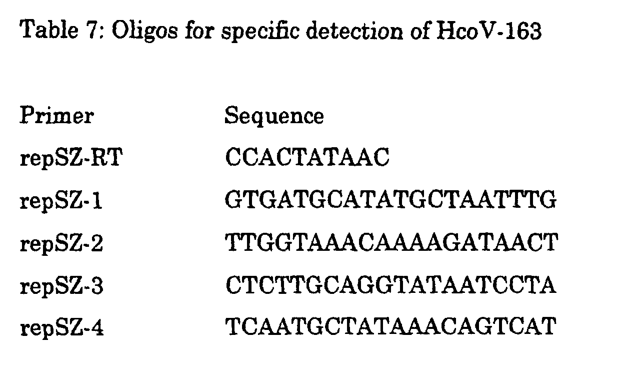

### Brief description of the drawings

Figure 1: cDNA-AFLP allows amplification of nucleic acids without any prior sequence information. Culture supernatants from CPE-positive and uninfected cells are subjected to the cDNA-AFLP procedure. Amplification products derived from the CPE-positive culture which are not present in the uninfected control sample are cloned and sequenced.
**Figure 2, LLC-MK2 cells infected with HCoV-SLA163.** Panel A and B are unstained cells while panel C and D are stained with haematoxilin eosin. The typical CPE of HCoV-SLA163 is shown in panel A and C. The control uninfected LLC-MK cells are shown in panel B and D.

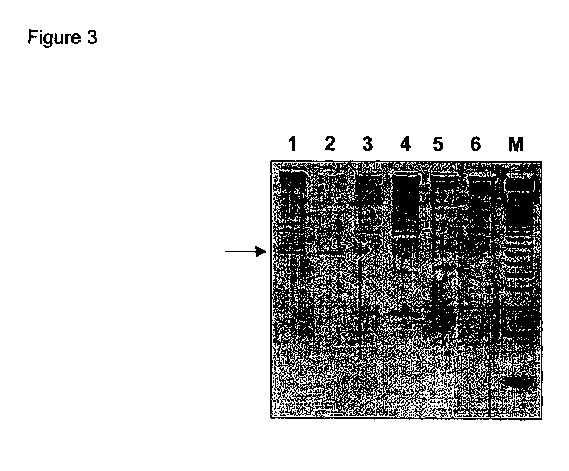

**Figure 3, VD-cDNA-AFLP PCR products visualized by Metaphor®** agarose gel electrophoreses. The PCR products of 1 (HinP I-G and Mse I-A) of 16 primer pair combinations used during the selective amplification step. Lanes 1 and 2: duplicate PCR product of virus culture SLA163; lanes 5 and 6 control supernatant of LLC-MK2 cells and in lane 7 and 8 the negative PCR control. Lanes M: 25bp molecular weight marker (InVitrogen). The arrow indicates a new coronavirus fragment that was excised out of gel and sequenced.

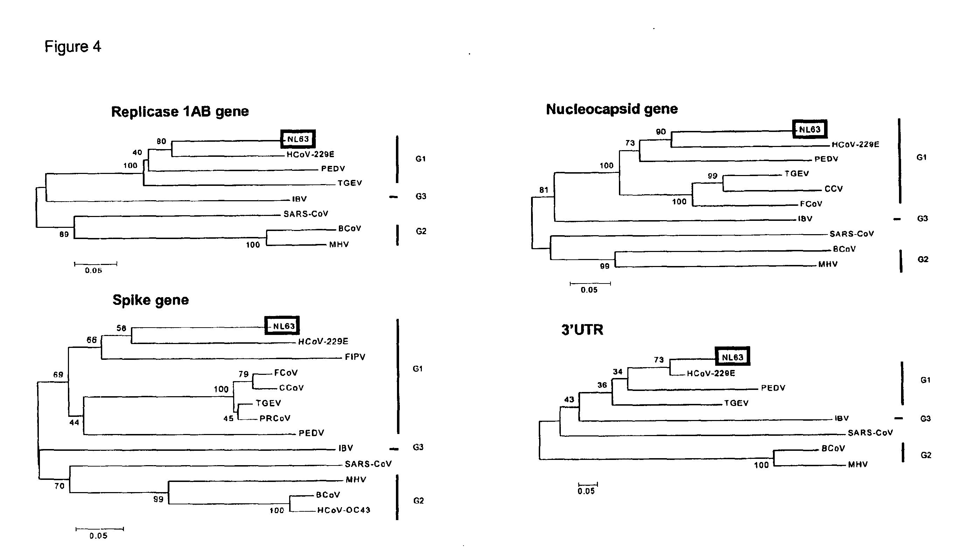

**Figure 4, Phylogenetic analysis of the HCoV-163 sequences.** G1, G2 and G3 denote the group 1, group 2 and group 3 coronavirus clusters. The Genbank accession number of the used sequences are: MHV (mouse hepatitis virus): AF201929; HCoV-229E: AF304460; PEDV (porcine epidemic diarrhea virus): AF353511; TGEV (transmissible gastroenteritis virus): AJ271965; SARS-CoV: AY278554; IBV (avian infectious bronchitis virus): NC_001451; BCoV (bovine coronavirus): NC_003045; FCoV (feline coronavirus): Y13921 and X80799; CCoV (canine coronavirus): AB105373 and A22732; PRCoV (porcine respiratory coronavirus) : M94097; FIPV (feline infectious peritonitis virus): D32044. Position of the HCoV-163 fragments compared to HCoV-229E (AF304460): Replicase 1AB gene: 15155-15361, 16049-16182, 16190-16315, 18444-18550, Spike gene: 22124-22266; Nucleocapsid gene: 25667-25882 and 25887-25957; 3'UTR: 27052-27123. Branch lengths indicate the number of substitutions per sequence.
Figure 5: Schematic representation of Coronavirus and the location of the 163-fragments listed in table 3.

### Reference List

1. Bachem, C.W., R.S. van der Hoeven, S.M. de Bruijn, D. Vreugdenhil, M. Zabeau, and R.G. Visser. 1996. Visualization of differential gene expression using a novel method of RNA fingerprinting based on AFLP: analysis of gene expression during potato tuber development. Plant J. 9:745-753.
2. Bestebroer, T.M., A.I.M. Bartelds, A.M. van Loon, H. Boswijk, K. Bijlsma, E.C.J. Claas, J.A.F.W. Kleijne, C.Verweij, M.W. Verweij-Uijterwaal, A.G. Wermenbol, and J. de Jong,. Virological NIVEL/RIVM-surveillance of respiratory virus infection in the season 1994/95. 245607002, 1-38. 1995. Bilthoven, RIVM. Virologische NIVEL/RIVM-surveillance van respiratoire virusinfecties in het seizoen 1994/95 RIVM. Ref Type: Report
3. Blondel, B., O. Akacem, R. Crainic, P. Couillin, and F. Horodniceanu. 1983. Detection by monoclonal antibodies of an antigenic determinant critical for poliovirus neutralization present on VP1 and on heat-inactivated virions. Virology 126:707-710.
4. Boom, R., C. J. Sol, M. M. Salimans, C. L. Jansen, P. M. Wertheim-van Dillen, and van der Noordaa J. 1990. Rapid and simple method for purification of nucleic acids. J. Clin. Microbiol. 28:495-503.
5. Kamur, S., Tamura, K., and Wei, M. Molecular Evolutionary Genetics Analysis (MEGA 2.0). 1993. Institute of Molecular Evolutionary Genetics, Pennsylvania State University, University Park. Ref Type: Computer Program
6. Kimura, M. 1980. A simple method for estimating evolutionary rates of base substitutions through comparative studies of nucleotide sequences. J. Mol. Evol. 16:111-120.
7. Kunkel, F. and G. Herrler. 1993. Structural and functional analysis of the surface protein of human coronavirus OC43. Virology 195:195-202.
8. Mounir, S., P. Labonte, and P. J. Talbot. 1993. Characterization of the nonstructural and spike proteins of the human respiratory coronavirus OC43: comparison with bovine enteric coronavirus. Adv. Exp. Med. Biol. 342:61-67.
9. Thompson, J. D., T. J. Gibson, F. Plewniak, F. Jeanmougin, and D. G. Higgins. 1997. The CLUSTAL_X windows interface: flexible strategies for multiple sequence alignment aided by quality analysis tools. Nucleic Acids Res. 25:4876-4882.
10. Van Den Hoogen, B. G., J. C. de Jong, J. Groen, T. Kuiken, R. de Groot, R. A. Fouchier, and A. D. Osterhaus. 2001. A newly discovered human pneumovirus isolated from young children with respiratory tract disease. Nat. Med. 7:719-724.
11. Wu, C. N., Y. C. Lin, C. Fann, N. S. Liao, S. R. Shih, and M. S. Ho. 2001. Protection against lethal enterovirus 71 infection in newborn mice by passive immunization with subunit VP1 vaccines and inactivated virus. Vaccine 20:895-904.
13. Almeida, J.D. and D.A. Tyrrell, The morphology of three previously uncharacterized human respiratory viruses that grow in organ culture. J Gen Virol 1, 175-178 (1967).
14. Thiel, V., J.Herold, , B. Schelle, and S.G. Siddell, Infectious RNA transcribed in vitro from a cDNA copy of the human coronavirus genome cloned in vaccinia virus. J Gen Virol 82, 1273-1281 (2001).
15. Hendley, J.O., H.B. Fishburne, and J.M. Gwaltney, Jr. Coronavirus infections in working adults. Eight-year study with 229 E and OC 43. Am Rev. Respir. Dis. 105, 805-811 (1972).
16. Mounir, S., P. Labonte, and P.J. Talbot, Characterization of the nonstructural and spike proteins of the human respiratory coronavirus OC43: comparison with bovine enteric coronavirus. Adv. Exp Med Biol 342, 61-67 (1993).
17. Kunkel, F. and G. Herrler, Structural and functional analysis of the surface protein of human coronavirus OC43. Virol. 195, 195-202 (1993).
18. Tyrrell, D.A.J. and M.L. Bynoe, Cultivation of novel type of common-cold virus in organ cultures. Br. Med J 1, 1467-1470 (1965).
19. Bradburne, A.F., M.L. Bynoe, and D.A. Tyrrell, Effects of a "new" human respiratory virus in volunteers. Br. Med J 3, 767-769 (1967).
20. Kapikian, A.Z. et al. Isolation from man of "avian infectious bronchitis virus-like" viruses (coronaviruses) similar to 229E virus, with some epidemiological observations. J Infect. Dis. 119, 282-290 (1969).
21. Ksiazek, T.G. et al. A novel coronavirus associated with severe acute respiratory syndrome. N Engl J Med 2003. May 15. ;348. (20):1953. -66. 348, 1953-1966 (2003).
22. Stohlman, S.A. and D.R. Hinton, Viral induced demyelination. Brain Pathol. 11, 92-106 (2001).
23. Jubelt, B. and J.R. Berger, Does viral disease underlie ALS? Lessons from the AIDS pandemic. Neurology 57, 945-946 (2001).
24. Shingadia, D., A. Bose, and R. Booy, Could a herpesvirus be the cause of Kawasaki disease? Lancet Infect. Dis. 2, 310-313 (2002).
25. Bachem, C.W. et al. Visualization of differential gene expression using a novel method of RNA fingerprinting based on AFLP: analysis of gene expression during potato tuber development. Plant J 9, 745-753 (1996).
26. Hamparian, V.V. Diagnostic procedures for viral, rickettsial and chlamydial infection. E.H. Lennette, and N.J. Schmidt, (eds.), pp. 5621979).
27. Marra, M.A. et al. The Genome sequence of the SARS-associated coronavirus. Science 2003. May 30. ;300. (5624. ):1399. -404. 300, 1399-1404 (2003).
28. McIntosh, K. et al. Coronavirus infection in acute lower respiratory tract disease of infants. J Infect. Dis. 130, 502-507 (1974).
29. Boivin, G. et al. Human metapneumovirus infections in hospitalized children. Emerg. Infect. Dis. 9, 634-640 (2003).
30. Rota, P.A. et al. Characterization of a novel coronavirus associated with severe acute respiratory syndrome. Science 2003. May 30. ;300. (5624. ):1394. - 9. 300, 1394-1399 (2003).
31. Bestebroer, T.M. et al. Virological NIVEL/RIVM-surveillance of respiratory virus infection in the season 1994/95. 245607002, 1-38. 1995. Ref Type: Report
32. van den Hoogen, B.G. et al. A newly discovered human pneumovirus isolated from young children with respiratory tract disease. Nat. Med 7, 719-724 (2001).
34. Earley, E. M. and K. M. Johnson. 1988. The lineage of Vero, Vero 76 and its clone C1008 in the United States., p. 26-29. In B. Simizu and T. Terasima (eds.), Vero cells: origin, properties and biomedical applications. Chiba Univ, Tokyo. 35. Kamur, S., K. Tamura, and M. Wei, Molecular Evolutionary Genetics Analysis (MEGA). (2.0). 1993. Institute of Molecular Evolutionary Genetics, Pennsylvania State University, University Park. Ref Type: Computer Program
36. Kimura, M. A simple method for estimating evolutionary rates of base substitutions through comparative studies of nucleotide sequences. J Mol Evol. 16, 111-120 (1980).
37 . Fouchier, R. A., T. M. Bestebroer, S. Herfst, K. L. Van Der, G. F. Rimmelzwaan, and A. D. Osterhaus. 2000. Detection of influenza A viruses from different species by PCR amplification of conserved sequences in the matrix gene. J. Clin. Microbiol. 38:4096-4101.
38. Nicaud, J. M., C. Madzak, B. P. van den, C. Gysler, P. Duboc, P. Niederberger, and C. Gaillardin. 2002. Protein expression and secretion in the yeast Yarrowia lipolytica. FEM. Yeast Res. 2:371-379

## Claims

1. An isolated or recombinant virus comprising a nucleic acid sequence as depicted in Table 3, or a functional part, derivative or analogue of said virus.

2. An isolated or recombinant virus comprising an amino acid sequence as depicted in Figure 1, or a functional part, derivative or analogue of said virus.

3. An isolated or recombinant virus comprising a nucleic acid sequence encoding an amino acid sequence as depicted in Figure 1, or a functional part, derivative or analogue of said virus.

4. An isolated or recombinant virus comprising an amino acid sequence which is more than 84 % homologous to at least part of a 163-2 amino acid sequence as depicted in Figure 1, said part having at least 30 amino acid residues.

5. An isolated or recombinant virus comprising an amino acid sequence which is more than 51 % homologous to at least part of a 163-4 amino acid sequence as depicted in Figure 1, said part having at least 30 amino acid residues.

6. An isolated or recombinant virus comprising a nucleic acid sequence which is more than 81 % homologous to at least part of a 163-9 nucleic acid sequence as depicted in Figure 1, said part having at least 30 nucleic acid residues.

7. An isolated or recombinant virus comprising an amino acid sequence which is more than 91 % homologous to at least part of a 163-10 amino acid sequence as depicted in Figure 1, said part having at least 30 amino acid residues.

8. An isolated or recombinant virus comprising an amino acid sequence which is more than 33 % homologous to at least part of a 163-11 amino acid sequence as depicted in Figure 1, said part having at least 30 amino acid residues.

9. An isolated or recombinant virus comprising an amino acid sequence which is more than 81 % homologous to at least part of a 163-14 amino acid sequence as depicted in Figure 1, said part having at least 30 amino acid residues.

10. An isolated or recombinant virus comprising an amino acid sequence which is more than 81 % homologous to at least part of a 163-15 amino acid sequence as depicted in Figure 1, said part having at least 20 amino acid residues.

11. An isolated or recombinant virus comprising an amino acid sequence which is more than 74 % homologous to at least part of a 163-18 amino acid sequence as depicted in Figure 1, said part having at least 30 amino acid residues.

12. An isolated or recombinant virus comprising a nucleic acid sequence which is at least 50 % homologous to at least part of a nucleic acid sequence as depicted in Table 3, said part having at least 50 nucleotides.

13. An isolated or recombinant virus or a functional part, derivative or analogue according to any one of claims 1-12, capable of inducing a HCoV-NL63-related disease.

14. A primer and/or probe, capable of specifically hybridizing to a nucleic acid of a virus or functional part, derivative or analogue according to any one of claims 1-13.

15. A primer and/or probe, capable of specifically hybridizing to a nucleic acid sequence as depicted in Table 3.

16. A primer and/or probe according to claim 14 or 15, which is capable of hybridizing to said nucleic acid under stringent conditions.

17. A primer and/or probe according to any one of claims 14-16, comprising a sequence as depicted in Table 7.

18. An isolated molecule capable of specifically binding a virus or functional part, derivative or analogue according to any one of claims 1-13.

19. An isolated molecule capable of specifically binding an amino acid sequence of a virus or functional part, derivative or analogue according to any one of claims 1-13.

20. An isolated molecule capable of specifically binding a nucleic acid sequence of a virus or functional part, derivative or analogue according to any one of claims 1-13.

21. An isolated molecule capable of specifically binding at least part of a nucleic acid sequence as depicted in Table 3.

22. An isolated molecule capable of specifically binding at least part of an amino acid sequence as depicted in Figure 1.

23. A molecule according to any one of claims 18-22 which is a proteinaceous molecule.

24. A method for producing a proteinaceous molecule capable of specifically binding a virus or functional part, derivative or analogue according to any one of claims 1-13 comprising:
- producing proteinaceous molecules capable of binding a virus or functional part, derivative or analogue according to any one of claims 1-13, and
- selecting a proteinaceous molecule that is specific for said virus.

25. An isolated or recombinant virus which is immunoreactive with a molecule according to any one of claims 18-23.

26. Use of a virus or functional part, derivative and/or analogue according to any one of claims 1-13 or 25 for detecting a molecule capable of specifically binding said virus in a sample.

27. Use of an amino acid sequence of a virus or functional part, derivative or analogue according to any one of claims 1-13 or 25, for detecting a molecule capable of specifically binding said virus or functional part, derivative and/or analogue in a sample.

28. Use according to claim 27, wherein said amino acid sequence comprises a sequence as depicted in Figure 1 or a functional part, derivative or analogue thereof.

29. Use according to any one of claims 26-28, wherein said molecule comprises a specific ligand and/or antibody of said virus.

30. Use of a primer and/or probe according to any one of claims 14-17, a molecule according to any one of claims 18-23, and/or a nucleic acid of a virus or functional part, derivative or analogue according to any one of claims 1-13 or 25, for detecting and/or identifying a HCoV-NL63 coronavirus in a sample.

31. Use according to claim 30 wherein said nucleic acid comprises a sequence as depicted in Table 3.

32. A virus or functional part, derivative or analogue according to any one of claims 1-13 or 25 for use as a vaccine or medicament.

33. A molecule according to any one of claims 18-23 for use as a vaccine or medicament.

34. Use of a virus or functional part, derivative or analogue according to any one of claims 1-13 or 25 for the preparation of a vaccine against a coronaviral genus related disease.

35. Use of a molecule according to any one of claims 18-23 for the preparation of a vaccine or medicament against a coronaviral genus related disease.

36. Use of a virus or functional part, derivative or analogue according to any one of claims 1-13 or 25, a molecule according to any one of claims 18-23, or a primer and/or probe according to any one of claims 14-17 for diagnosis of a coronaviral genus related disease.

37. Use according to any one of claims 34-36 wherein said coronaviral genus related disease comprises an HCoV-NL63 coronavirus related disease.

38. A vaccine comprising a virus or functional part, derivative or analogue according to any one of claims 1-13 or 25 and/or a molecule according to any one of claims 18-23.

39. A medicament comprising a virus or functional part, derivative or analogue according to any one of claims 1-13 or 25 and/or a molecule according to any one of claims 18-23.

40. A vaccine or medicament according to claim 38 or 39 for at least in part preventing and/or treating an HCoV-NL63-related disease.

41. A method for detecting a virus or functional part, derivative or analogue according to any one of claims 1-13 or 25 in a sample, comprising hybridizing and/or amplifying a nucleic acid of said virus or functional part, derivative or analogue with a primer and/or probe according to any one of claims 14-17 and detecting hybridized and/or amplified product.

42. A diagnostic kit comprising a virus or functional part, derivative or analogue according to any one of claims 1-13 or 25, a molecule according to any one of claims 18-23, and/or a primer/probe according to any one of claims 14-17.

43. A method for treating an individual suffering from, or at risk of suffering from, an HCoV-NL63 related disease, comprising administering to said individual a vaccine or medicament according to any one of claims 38-40.

44. A method for determining whether an individual suffers from an HCoV-NL63 related disease, comprising obtaining a sample from said individual and detecting a HCoV-NL63 virus or functional part, derivative or analogue thereof in said sample with a method according to claim 41 or a diagnostic kit according to claim 42.

45. An isolated or recombinant nucleic acid encoding a virus or functional part, derivative and/or analogue according to any one of claims 1-13 or 25.

46. A nucleic acid according to claim 45, comprising at least a functional part of a sequence as depicted in Table 3.

47. An amino acid sequence encoded by a nucleic acid according to claim 45 or 46.

48. An amino acid sequence according to claim 47, comprising at least a functional part of a sequence as depicted in Figure 1.

49. A proteinaceous molecule capable of specifically binding HCoV-NL63, obtainable by a method according to claim 24.

50. Use of a proteinaceous molecule according to claim 49 in a vaccine or a diagnostic method for the detection of HCoV-NL63.
